# EUROPEAN PATENT APPLICATION

(11) **EP 4 810 107 A1**
(43) Date of publication of application: **23.09.2026**
(21) Application number: 24891702.3
(22) Date of filing: 06.11.2024
(51) Int. Cl.: A61N 1/40, A61N 1/06, A61N 1/32, A61N 1/36

(54) **TIP MODULE FOR SKIN TREATMENT DEVICE USING RF ENERGY, SKIN TREATMENT DEVICE USING RF ENERGY INCLUDING TIP MODULE, AND SKIN TREATMENT METHOD USING RF ENERGY**

(30) Priority: 16.11.2023 KR 20230158949
(71) Applicant: Lutronic Corporation, Goyang-si, Gyeonggi-do 10534 (KR)
(72) Inventor: KO, Kwangchon, Goyang-si, Gyeonggi-do 10394 (KR)
(74) Representative: Algemeen Octrooi- en Merkenbureau B.V.
(86) International application number: PCT/KR2024/017357
(87) International publication number: WO 2025/105755

(57) **Abstract**

The present disclosure relates to a tip module that divides electrodes into a central region, an intermediate region, and an outer region and minimizes the size of the individual electrodes in the outer region to uniformize the overall current density; a skin treatment device using RF energy including the same; and a skin treatment method using RF energy.

## Description

### CROSS REFERENCE TO RELATED APPLICATION

This application claims priority from Republic of Korea Patent Application No. 10-2023-0158949, filed on November 16, 2023, which is hereby incorporated by reference in its entirety.

### BACKGROUND

### Field

The present disclosure relates to a tip module for a skin treatment device using RF energy that can minimize edge effects and achieve optimal therapeutic effects, a skin treatment device using RF energy including the same, and a skin treatment method using RF energy.

### Related Art

Devices that transmit RF energy to tissue for therapeutic purposes have been developed in various ways. Recently, devices have been developed that use RF energy to cause appropriate degeneration of the skin and exert a skin treatment effect through tissue regeneration.

As a method for heating skin tissue, a method of heating by transmitting RF energy is widely used. Among devices that treat the skin using RF energy, a treatment using electrodes in contact with the skin surface has also been developed to relieve pain and minimize side effects. However, when RF energy is transmitted using such conventional contact electrodes, there is a problem that the current is concentrated at the edge of the electrode, which may cause excessive damage to the tissue.

### (Prior Art Document)

### (Patent Document)

US Patent No. 8,496,654

### SUMMARY

The present disclosure provides a tip module for a skin treatment device using RF energy that can minimize side effects due to edge effects when treating skin tissue by transmitting RF energy with a conventional non-invasive electrode, a skin treatment device using RF energy including the same, and a skin treatment method using RF energy.

In accordance with an aspect of the present disclosure, there is provided a tip module for a skin treatment device using RF energy, comprising: a housing having an open side; a substrate provided on the open side of the housing; and an electrode provided on an outer surface of the substrate, wherein the electrode is divided into a central electrode, an intermediate electrode and an outer electrode, the intermediate electrode is divided into a plurality of intermediate electrode parts, and the outer electrode is divided into a plurality of outer electrode parts.

In this case, the electrodes of the tip module may form a spider pattern. As an example, the electrode may be divided into multiple electrodes along lines reminiscent of a spider web.

Meanwhile, when transmitting RF energy to skin tissue, a current density (A/mm²) of the RF energy in the central electrode, each intermediate electrode part, and each outer electrode part may be maintained within a preset range.

Meanwhile, an area of each intermediate electrode part may be configured to be larger than an area of each outer electrode part.

Meanwhile, the intermediate electrode and the outer electrode may be formed along concentric round paths of different sizes.

Meanwhile, the intermediate electrode may be divided into the intermediate electrode parts by a plurality of first cutting lines formed in a width direction of the rounded path, and the outer electrode may be divided into the outer electrode parts by a plurality of second cutting lines formed in the width direction of the rounded path.

Meanwhile, the first cutting line may be formed continuously with the second cutting line.

Further, the first cutting line and the second cutting line are formed radially.

Furthermore, the central electrode, the intermediate electrode parts, and the outer electrode parts may be configured to simultaneously transmit the RF energy by an external control unit.

Meanwhile, each of the central electrode, the intermediate electrode parts, and the outer electrode parts may be configured to independently transmit the RF energy by an external control unit.

In accordance with another aspect of the present disclosure, there is provided a skin treatment device using RF energy, comprising: a main body; an RF generator provided in the main body and configured to generate RF energy; a control unit provided in the main body and configured to control the RF energy; a handpiece connected to the main body and configured to be held by a user; and a tip module detachably provided at an end of the handpiece, wherein the tip module includes: a housing having an open side; a substrate provided on the open side of the housing; and an electrode provided on an outer surface of the substrate, and wherein the electrode is divided into a central electrode, an intermediate electrode and an outer electrode, the intermediate electrode is divided into a plurality of intermediate electrode parts, and the outer electrode is divided into a plurality of outer electrode parts.

In accordance with still another aspect of the present disclosure, there is provided a skin treatment method using RF energy, comprising: contacting an RF electrode to skin tissue; transmitting RF energy to the tissue through the RF electrode; and heating the tissue with the RF energy to treat the tissue, wherein a transmission surface that transmits the RF energy to the skin tissue is divided into a central region, an intermediate region, and an outer region, the intermediate region is divided into a plurality of intermediate region parts, the outer region is divided into a plurality of outer region parts, and the transmitting of the RF energy is performed by maintaining a current density of the RF energy within a preset range in each of the central region part, the intermediate region parts, and the outer region parts.

Meanwhile, an area of each intermediate region part may be larger than an area of each outer region part.

Further, the intermediate region and the outer region may be formed along concentric round paths of different sizes.

Meanwhile, the intermediate region may be divided into the intermediate region parts by a plurality of first cutting lines formed in a width direction of the rounded path.

Further, the outer region may be divided into the outer region parts by a plurality of second cutting lines formed in the width direction of the rounded path.

Meanwhile, the first cutting line may be formed continuously with the second cutting line.

Further, the first cutting line and the second cutting line may be formed radially.

Meanwhile, an area of a region through which a line extending from a center point to a perimeter of the transmission surface passes may be in a relationship of the central region > the intermediate region part > the outer region part.

Meanwhile, the transmitting of the RF energy may be performed by simultaneously transmitting the RF energy to the central region, the intermediate region parts, and the outer region parts.

In addition, the transmitting of the RF energy may be performed by independently transmitting the RF energy to the central region, the intermediate region parts, and the outer region parts.

The tip module for a skin treatment device using RF energy according to the present disclosure, the skin treatment device using RF energy including the same, and the skin treatment method using RF energy enable optimized skin treatment by minimizing edge effects.

### BRIEF DESCRIPTION OF THE DRAWINGS

FIG. 1 is a perspective view of a tip module for a skin treatment device using RF energy according to one embodiment of the present disclosure.
FIG. 2 is a partial cross-sectional view of an end portion of the tip module in FIG. 1.
FIG. 3 is a plan view showing electrodes in a first embodiment.
FIG. 4 is a diagram showing the tendency of size change of the electrodes in the first embodiment.
FIG. 5 is a conceptual diagram when heating tissue using electrodes divided into uniform sizes.
FIG. 6 is a conceptual diagram when heating tissue using the tip module according to the present disclosure.
FIG. 7 is a perspective view of a treatment device using RF energy, which is a second embodiment of the present disclosure.
FIG. 8 is a flowchart of a skin treatment method using RF energy, which is a third embodiment of the present disclosure.
FIG. 9 is a flowchart showing each step in detail in the third embodiment.
FIG. 10 is a diagram showing the concept of an energy transmission region according to the third embodiment.
FIG. 11 is a flowchart according to a modification of the third embodiment.

### DESCRIPTION OF EXEMPLARY EMBODIMENTS

Hereinafter, a tip module for a skin treatment device using RF energy according to an embodiment of the present disclosure, a skin treatment device including the same, and a skin treatment method using RF energy will be described in detail with reference to the accompanying drawings. In the following description of the embodiments, respective components may be referred to by different names in the art. However, if there is functional similarity and identity between them, they can be considered as equivalent configurations even if modification is applied thereto. In addition, reference numerals are given to the respective components for convenience of explanation. However, the contents shown in the drawings in which these reference numerals are given do not limit the respective components to the scope within the drawings. Likewise, even when an embodiment in which the configurations in the drawings are partially modified is adopted, if there is functional similarity and identity, they can be considered as equivalent configurations. In addition, if a component is recognized as obvious to be included in light of the level of ordinary skill in the art, description thereof will be omitted.

In the present specification, treatment means heating skin tissue to exert the effect of improving Wrinkles, Tone and Textural Changes, Scars and Acne Scarring, Sagging mucosa, Overall Rejuvenation, Hyperhidrosis, laxity, lifting, tightening, Fat reduction, etc.

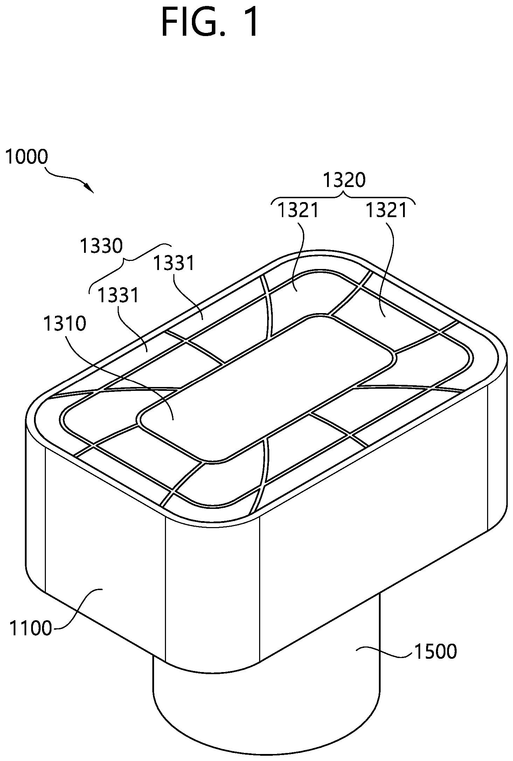

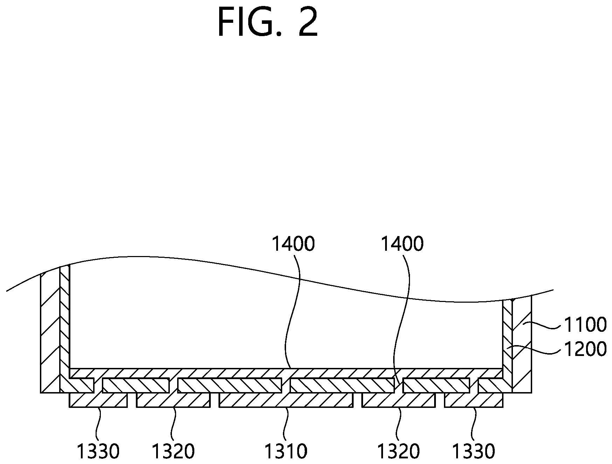

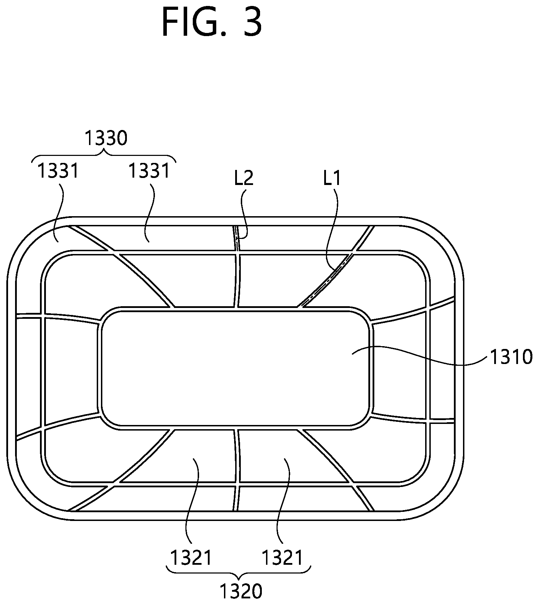

FIG. 1 is a perspective view of a tip module for a skin treatment device using RF energy according to one embodiment of the present disclosure, FIG. 2 is a partial cross-sectional view of an end portion of the tip module in FIG. 1, and FIG. 3 is a plan view showing electrodes in a first embodiment.

Referring to FIG. 1, a tip module 1000 for a skin treatment device using RF energy according to one embodiment of the present disclosure may include a housing 1100, a connection portion 1500, a substrate 1200, and an electrode.

The housing 1100 is configured to secure the electrode and the substrate 1200. The housing 1100 may have an inner space, and an open side. The connection portion 1500 may be provided at the rear of the housing 1100. The connection portion 1500 may be configured to be detachably attached to a handpiece of the treatment device using RF energy, which will be described later. The connection portion 1500 may be mechanically coupled to the handpiece, and also electrically connected to the handpiece.

The substrate 1200 is configured to be provided with a circuit for transmitting RF energy to a plurality of electrodes. In addition, the substrate 1200 serves as a base on which electrodes may be fixed. Meanwhile, although not shown, a plurality of sensors, for example, temperature sensors, may be provided on the substrate 1200 to measure temperature. The electrodes may be connected at a rear surface of the substrate 1200 through a conductive portion 1400 that penetrates the substrate 1200.

The electrode may include a plurality of electrodes and may be configured to be in contact with the skin to transmit RF energy to the skin. The electrode may be configured to be capacitively coupled to the skin to heat a deep portion of the skin by RF energy.

The electrode is provided on an end portion of the tip module 1000 and may be provided on one open side of the housing 1100. The electrode may be disposed in a predetermined area on the end portion of the tip module 1000.

Referring to FIG. 3, in a first embodiment according to the present disclosure, electrodes may be disposed in divided regions of the tip module 1000. An energy transmission region is defined at the end portion of the tip module 1000, and the energy transmission region may be defined as a region where RF energy is transmitted by a central electrode 1310, an intermediate electrode 1320, and an outer electrode 1330.

The central electrode 1310 is disposed in a central portion of a contact surface where the tip module 1000 is in contact with the skin. The outer electrode 1330 is provided at an outer periphery of the contact surface of the tip module 1000. The intermediate electrode 1320 may be provided between the central electrode 1310 and the outer electrode 1330.

That is, on a plane, the intermediate electrode 1320 may be formed to have a predetermined width along a rounded path around the central electrode 1310. In addition, the outer electrode 1330 may be formed to have a preset width along a rounded path around the intermediate electrode 1320.

That is, the intermediate electrode 1320 and the outer electrode 1330 may be formed along the concentric rounded paths with different radii.

The rounded path may generally follow a rectangular shape with rounded edges on the plane. However, this is only an example, and when the curvature of the edge on the plane increases, the rounded path may become a stadium path.

The intermediate electrode 1320 may be divided into a plurality of intermediate electrode parts 1321 by a plurality of first cutting lines L1 along the width direction. In addition, the outer electrode 1330 may be divided into a plurality of outer electrode parts 1331 by a plurality of second cutting lines L2 along the width direction.

In this case, the first cutting line L1 and the second cutting line L2 may be curved. In addition, the first cutting line L1 and the second cutting line L2 may be formed continuously. As an example, the first cutting line L1 and the second cutting line L2 may be formed along a sinusoidal wave path. Further, the first cutting line L1 and the second cutting line L2 may be formed repeatedly at predetermined intervals along the rounded path. As a result, the first cutting line L1 and the second cutting line L2 may appear radially around the central electrode 1310 on the contact surface.

The first cutting line L1 and the second cutting line L2 may be a gap between the intermediate electrode parts 1321 or the outer electrode parts 1331. In addition, an insulator may be provided along the first cutting line L1 and the second cutting line L2.

As described above, each of the intermediate electrode part 1321 and the outer electrode part 1331 is defined by the first cutting line L1 or the second cutting line L2 and the rounded path. Accordingly, the shape of each of the intermediate electrode part 1321 and the outer electrode part 1331 may include at least one curve.

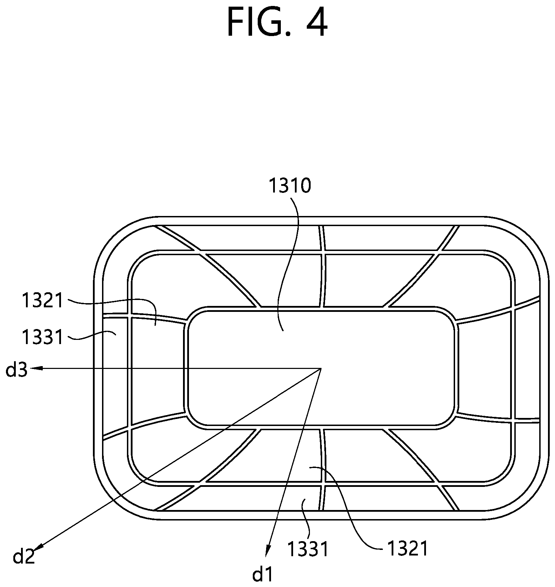

FIG. 4 is a diagram showing the tendency of size change of the electrodes in the first embodiment.

Referring to FIG. 4, the sizes of the electrodes may appear different in the energy transmission region. An imaginary line may be defined to explain the tendency of electrode size changes depending on location. For example, in case that lines d1, d2, d3 extending from the center to the outside of the contact surface are defined, when considering the sizes of the electrodes through which these lines pass, the central electrode 1310 is the largest size, followed by the intermediate electrode part 1321, and then the outer electrode part 1331.

In other words, the sizes of the electrodes may become smaller from the center to the outside.

Meanwhile, the total area of the divided electrodes may be in the relationship of intermediate electrode 1320 > outer electrode 1330. That is, on the contact surface, the total area of the intermediate electrode parts 1321 may be larger than the total area of the outer electrode parts 1331.

As described above, the intermediate electrode 1320 is divided into intermediate electrode parts 1321, and the outer electrode 1330 is divided into outer electrode parts 1331. Therefore, no matter which direction is selected from the center of the contact surface, for example, when the direction along any of the lines d1, d2, d3 is selected, the size of the electrode is in the relationship of central electrode 1310 > intermediate electrode 1320 > outer electrode 1330.

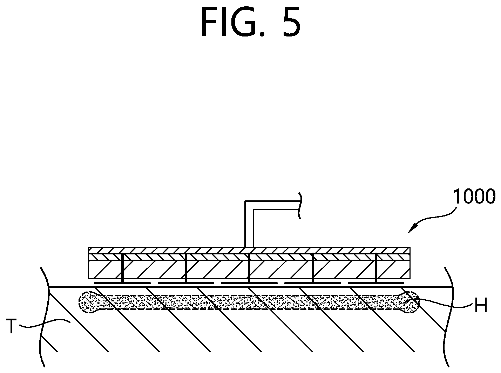

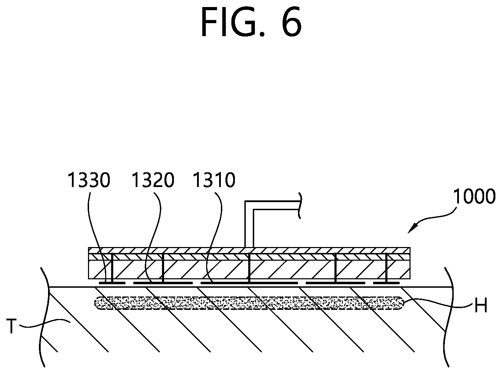

Hereinafter, with reference to FIGS. 5 and 6, the heating zone when transmitting RF energy using the first embodiment according to the present disclosure will be described.

FIG. 5 is a conceptual diagram when heating tissue using electrodes divided into uniform sizes. The electrodes shown in FIG. 5 can be viewed as a conventional technology, and RF energy can be transmitted by contacting the skin with the electrodes divided into equal sizes. As a result, a deeper portion of the tissue can be heated by RF energy. However, excessive current flows in the portion corresponding to the edge due to the edge effect, resulting in an increase in the heating zone. Even if this RF energy transmission tendency is maintained for several to tens of µs, the skin may suffer excessive and irreparable damage.

FIG. 6 is a conceptual diagram when heating tissue using the tip module 1000 according to the present disclosure.

Referring to FIG. 6, when transmitting RF energy using the tip module 1000 according to the present disclosure, the central electrode 1310 transmits RF energy to a large region, followed by the intermediate electrode part 1321, and then the outer electrode part 1331. As described above, the area of the electrode is in the relationship of central electrode 1310 > intermediate electrode part 1321 > outer electrode part 1331. Since each electrode is capacitively coupled to skin tissue, the amount of current generated by RF energy may vary depending on the area of the electrode. In other words, the larger the electrode area, the lower the current caused by RF energy. Therefore, the electrode in the center portion can be configured to be the largest, and the outer electrode part 1331, where current is concentrated due to the edge effect, can be configured to be the smallest. In this way, by configuring the size of the single electrode to become gradually smaller as it goes outward, the current density (A/mm²) can be maintained within a certain range. In other words, the area of the outer electrode part 1331 is configured to be small to reduce the amount of current in the tissue in contact with the edge portion, and even if an edge effect occurs, there is no significant deviation in temperature of the heated portion H in the tissue.

Meanwhile, although FIG. 6 illustrates a configuration in which RF energy is applied equally to the central electrode 1310, the intermediate electrode part 1321, and the outer electrode part 1331, each electrode and part may be independently applied with RF energy. In this case, the central electrode 1310, each intermediate electrode part 1321, and each outer electrode part 1331 can independently adjust RF energy by a control unit. As an example, the control unit may control the power or time of RF energy applied to the central electrode 1310 and each part 1321 and 1331.

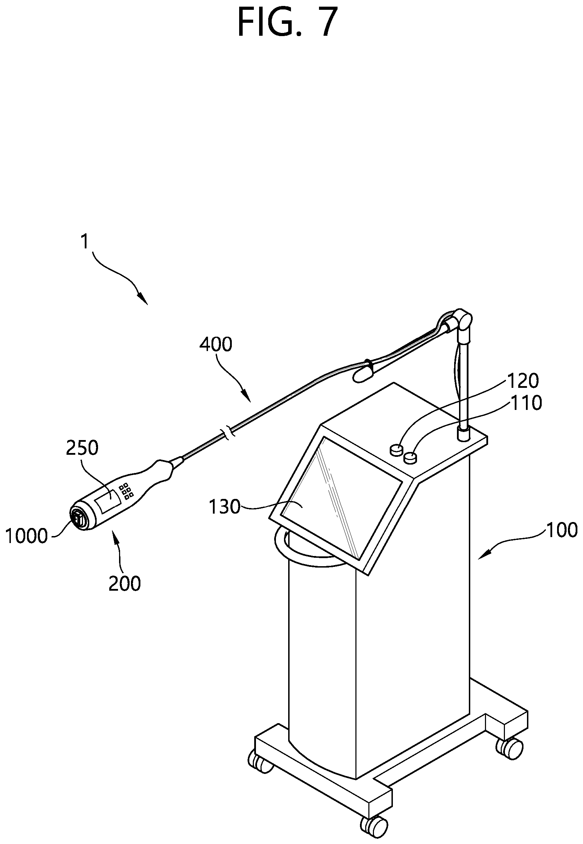

Hereinafter, a treatment device using RF energy, which is a second embodiment according to the present disclosure, will be described with reference to FIG. 7.

FIG. 7 is a perspective view of a treatment device 1 using RF energy, which is the second embodiment of the present disclosure.

Referring to FIG. 7, the skin treatment device 1 using RF energy with a duplicate treatment prevention function according to one embodiment of the present disclosure may include a main body 100, a handpiece 200 that a user can hold to perform treatment, a connection portion 400 that connects the main body 100 and the handpiece 200, and a tip module 1000 configured to be detachably attached at an end of the handpiece 200.

An RF generator (not shown), an RF adjustment unit (not shown), and a control unit (not shown) may be provided inside the main body 100. As described above, the control unit generates a control input to control the RF generator according to a sensing value input from a sensor unit. In this case, the frequency of the RF energy may be adjusted depending on a patient's constitution, treatment purpose, treatment region, etc.

On an outer surface of the main body 100, a power on/off switch 110, a frequency control lever 120 that can adjust the frequency of the RF energy generated from the RF generator, and a touch screen 130 which displays various information including the operation details of the treatment device 1 and treatment information, and through which a user can input commands.

The handpiece 200 is connected to the main body 100 by the connection portion 400. The handpiece 200 is configured to be gripped and used by the user. The tip module 1000 may be provided at the distal end of the handpiece 200. The user may press the tip module 1000 against the patient's skin and manipulate the handpiece 200 to transmit RF energy to the tissue. A display unit 250 may be provided on one side of the handpiece to display information related to treatment or operation of the treatment device 1 when the user holds and manipulates the handpiece 200.

The handpiece 200 transmits RF energy generated from the RF generator of the main body 100 to the plurality of electrodes provided at the end of the tip module 1000 through the connection portion 400. In addition, the handpiece 200 is configured to measure the temperature through a plurality of temperature sensors provided in the tip module 1000 when RF energy is transmitted to the tissue and transmit it to the control unit. The user may place the tip module 1000 on the handpiece 200 in close contact with the skin and perform an input that transmits RF energy. After RF energy is transmitted to the skin tissue according to the user's input, the user moves the position of the handpiece 200 and then performs the input to transmit the RF energy to the tissue again. The user can perform treatment on a large region of tissue, such as the entire face, by repeating this process several to hundreds of times.

Meanwhile, the present embodiment may include the tip module 1000 described with reference to FIGS. 1 to 6. In addition, in the present embodiment, the user can replace the tip module 1000 when necessary.

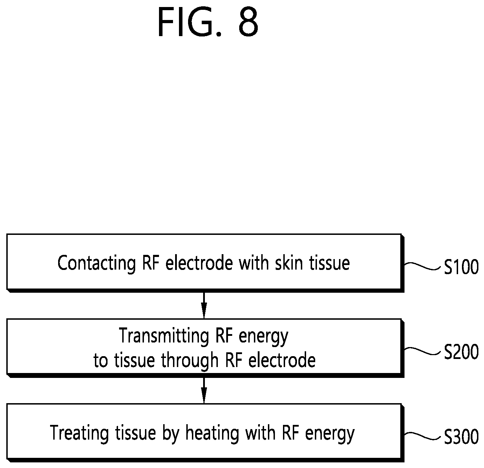

Hereinafter, a skin treatment method using RF energy, which is a third embodiment of the present disclosure, will be described with reference to FIGS. 8 to 10.

FIG. 8 is a flowchart of the skin treatment method using RF energy, which is the third embodiment of the present disclosure.

The skin treatment method using RF energy, according to the third embodiment of the present disclosure, may include a step of contacting the RF electrode with skin tissue (S100), a step of transmitting RF energy to the tissue through the RF electrode (S200), and a step of treating the tissue with the RF energy (S300).

The step of contacting the electrode with the skin tissue (S100) includes contacting the end of the handpiece on which the electrode is arranged to the affected region.

The step of transmitting RF energy to the tissue through the RF electrode (S200) may be performed using the electrode provided in the tip module described in the first embodiment above.

The step of Treating tissue by heating with RF energy (S300) corresponds to the step of heating the skin when transmitting RF energy using the tip module, which is the first embodiment. In this case, the region on the plane where RF energy is transmitted may be defined as a 'transmission surface' of the energy. The transmission surface may be divided into a central region, an outer region, and an intermediate region.

The intermediate region may be formed with a predetermined width along a rounded path around the central region. The outer region may be formed with a preset width along a rounded path around the intermediate region. That is, with the central region as the center, the intermediate region and the outer region may be formed along the concentric rounded paths of different sizes.

The intermediate region may be divided into a plurality of intermediate region parts along the rounded path. The intermediate region may be divided by a plurality of first cutting lines formed in the width direction of the rounded path. The outer region may be divided into a plurality of outer region parts along the rounded path. The outer region may be divided by a plurality of second cutting lines. The first cutting line and the second cutting line may be formed radially on the transmission surface and may also be formed continuously with each other.

In this case, since the intermediate region is divided into intermediate region parts by the plurality of first cutting lines formed in the width direction in the rounded path, the central region may have a larger area than the intermediate region part. In addition, the intermediate region part may have a larger area than the outer region part.

Further, the total area of the intermediate region parts may be greater than the total area of the outer region parts.

In other words, the central region may be defined as a region where RF energy is transmitted by the central electrode of the tip module, the intermediate region part by the intermediate electrode part, and the outer region part by the outer electrode part. Additionally, the size of each region and region part may follow the size of the central electrode and parts provided in the electrode.

In this way, the edge effect can be minimized by dividing the RF energy transmission surface and forming the outer part to the smallest size.

In addition, according to the third embodiment of the present disclosure, the current density (A/mm²) can be maintained in a certain range in the central region, the intermediate region, and the outer region, which enables uniform heating and ultimately uniform treatment of tissue.

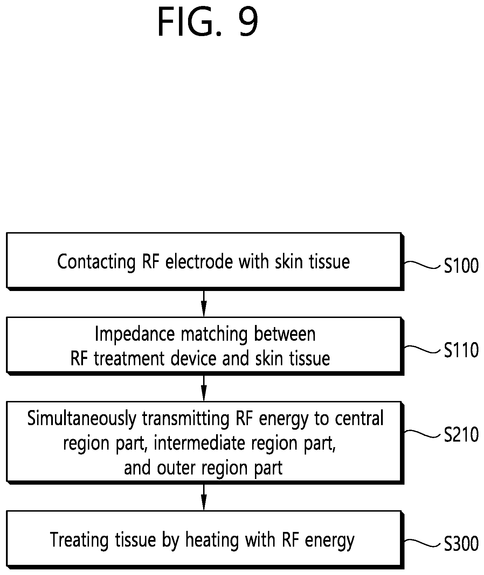

FIG. 9 is a flowchart showing each step in detail in the third embodiment.

Referring to FIG. 9, the third embodiment may further include a step S110 of impedance matching between the RF treatment device and the skin tissue after performing the step S100 of contacting the RF electrode with the skin tissue. The RF electrode and the skin tissue may be capacitively coupled, and the impedance matching may be performed to increase heating efficiency.

In addition, in the third embodiment, the step of transmitting RF may be performed by simultaneously transmitting RF energy to the central region part, the intermediate region part, and the outer region part (S210). In other words, RF energy may be simultaneously applied to the respective divided regions to heat them simultaneously. Even in this case, it is possible to prevent strong current due to RF energy from flowing in the outer region.

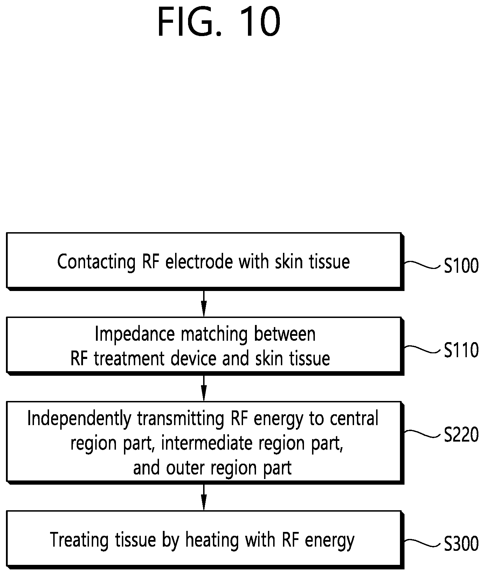

FIG. 10 is a diagram showing the concept of an energy transmission region according to the third embodiment.

Referring to FIG. 10, RF energy may be transmitted into the tissue T through a central region 2100, an intermediate region 2200, and an outer region 2300 on the surface of the skin tissue. In this case, RF energy may be transmitted to the intermediate region through a plurality of intermediate region parts. In addition, RF energy may be transmitted to the outer region through a plurality of outer region parts. The heated area H in the tissue may vary depending on the frequency of the RF energy. As an example, skin tissue may be comprised of Epidermis, Dermis (Papillary dermis, Reticular dermis), Hypodermis, and fat layer from the surface, and the frequency may be varied to control the depth of heating. Even if the frequency of such RF energy is adjusted, the edge effect can be minimized according to the present disclosure, thereby preventing excessive damage to the tissue and achieving optimal therapeutic effects.

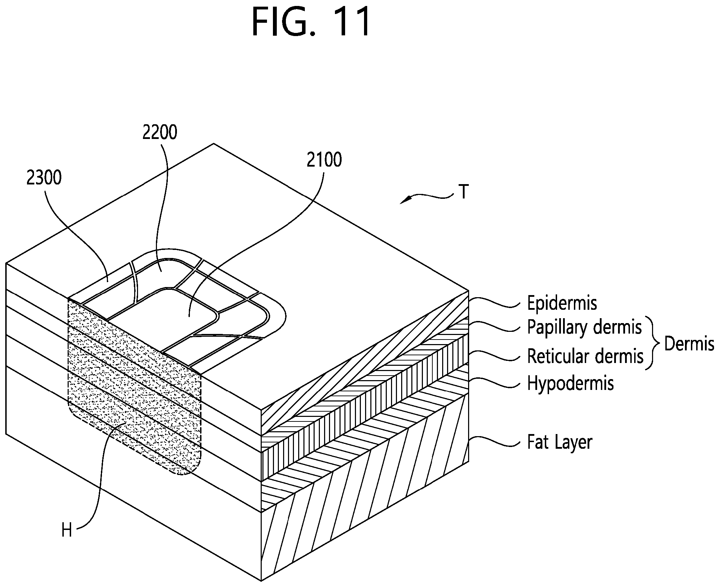

FIG. 11 is a flowchart according to a modification of the third embodiment.

Referring to FIG. 11, in the third embodiment, the RF transmitting step may independently transmit RF energy to the central region, the intermediate region part, and the outer region part (S220). That is, contrary to the example described in FIG. 9, RF energy can be transmitted independently to each region. In this case, the power and time of RF energy applied to the central region, the intermediate region part, and the outer region part can be controlled independently, thereby enabling precise control in terms of the total energy transmitted. Therefore, the heated portion in the tissue can be heated uniformly, and control such as reducing the power of RF energy applied to the outer region part can be performed in consideration of the occurrence of edge effects.

As described above, the tip module for the skin treatment device using RF energy and the skin treatment method using RF energy according to the present disclosure can minimize edge effects by the shape of the electrode in a capacitive coupling state, thereby preventing excessive damage to skin tissue and enabling optimized skin treatment.

### (Description of Reference Numerals)

- 1000:: tip module
- 1100:: housing
- 1200:: substrate
- 1310:: central electrode
- 1320:: intermediate electrode
- 1321:: intermediate electrode part
- 1330:: outer electrode
- 1331:: outer electrode part
- 1400:: conductive portion
- 1500:: connection portion
- 2100:: central region
- 2200:: intermediate region
- 2300:: outer region

## Claims

1. A tip module for a skin treatment device using RF energy, comprising:
a housing having an open side;
a substrate provided on the open side of the housing; and
an electrode provided on an outer surface of the substrate,
wherein the electrode is divided into a central electrode, an intermediate electrode and an outer electrode,
the intermediate electrode is divided into a plurality of intermediate electrode parts, and
the outer electrode is divided into a plurality of outer electrode parts.

2. The tip module of claim 1, wherein when transmitting RF energy to skin tissue, a current density of the RF energy in the central electrode, each intermediate electrode part, and each outer electrode part is maintained within a preset range.

3. The tip module of claim 2, wherein an area of each intermediate electrode part is larger than an area of each outer electrode part.

4. The tip module of claim 3, wherein a total area of the intermediate electrode parts is larger than a total area of the outer electrode parts.

5. The tip module of claim 3, wherein the intermediate electrode and the outer electrode are formed along concentric round paths of different sizes.

6. The tip module of claim 5, wherein the intermediate electrode is divided into the intermediate electrode parts by a plurality of first cutting lines formed in a width direction of the rounded path, and
the outer electrode is divided into the outer electrode parts by a plurality of second cutting lines formed in the width direction of the rounded path.

7. The tip module of claim 6, wherein the first cutting line is formed continuously with the second cutting line.

8. The tip module of claim 7, wherein the first cutting line and the second cutting line are formed radially.

9. The tip module of claim 7, wherein the central electrode, the intermediate electrode parts, and the outer electrode parts are configured to simultaneously transmit the RF energy by an external control unit.

10. The tip module of claim 7, wherein each of the central electrode, the intermediate electrode parts, and the outer electrode parts is configured to independently transmit the RF energy by an external control unit.

11. A skin treatment device using RF energy, comprising:
a main body;
an RF generator provided in the main body and configured to generate RF energy;
a control unit provided in the main body and configured to control the RF energy;
a handpiece connected to the main body and configured to be held by a user; and
a tip module detachably provided at an end of the handpiece,
wherein the tip module includes:
a housing having an open side;
a substrate provided on the open side of the housing; and
an electrode provided on an outer surface of the substrate, and
wherein the electrode is divided into a central electrode, an intermediate electrode and an outer electrode,
the intermediate electrode is divided into a plurality of intermediate electrode parts, and
the outer electrode is divided into a plurality of outer electrode parts.

12. A skin treatment method using RF energy, comprising:
contacting an RF electrode to skin tissue;
transmitting RF energy to the tissue through the RF electrode; and
heating the tissue with the RF energy to treat the tissue,
wherein a transmission surface that transmits the RF energy to the skin tissue is divided into a central region, an intermediate region, and an outer region,
the intermediate region is divided into a plurality of intermediate region parts,
the outer region is divided into a plurality of outer region parts, and
the transmitting of the RF energy is performed by maintaining a current density of the RF energy within a preset range in each of the central region, the intermediate region parts, and the outer region parts.

13. The skin treatment method of claim 12, wherein an area of each intermediate region part is larger than an area of each outer region part.

14. The skin treatment method of claim 13, wherein a total area of the intermediate region parts is larger than a total area of the outer region parts.

15. The skin treatment method of claim 13, wherein the intermediate region and the outer region are formed along concentric round paths of different sizes.

16. The skin treatment method of claim 15, wherein the intermediate region is divided into the intermediate region parts by a plurality of first cutting lines formed in a width direction of the rounded path.

17. The skin treatment method of claim 16, wherein the outer region is divided into the outer region parts by a plurality of second cutting lines formed in the width direction of the rounded path.

18. The skin treatment method of claim 17, wherein the first cutting line is formed continuously with the second cutting line.

19. The skin treatment method of claim 17, wherein the first cutting line and the second cutting line are formed radially.

20. The skin treatment method of claim 19, wherein an area of a region through which a line extending from a center point to a perimeter of the transmission surface passes is in a relationship of the central region > the intermediate region part > the outer region part.

21. The skin treatment method of claim 19, wherein the transmitting of the RF energy is performed by simultaneously transmitting the RF energy to the central region, the intermediate region parts, and the outer region parts.

22. The skin treatment method of claim 19, wherein the transmitting of the RF energy is performed by independently transmitting the RF energy to the central region, the intermediate region parts, and the outer region parts.
